# EUROPEAN PATENT SPECIFICATION

(11) **EP 3 302 353 B1**
(45) Date of publication and mention of the grant of the patent: **26.08.2020**
(21) Application number: 16800479.4
(22) Date of filing: 06.05.2016
(51) Int. Cl.: A61L 27/36, A61L 27/38, A61L 27/54, A61K 35/12, A61K 35/30, A61F 2/02, C12N 5/079, C12N 5/0793

(54) **DECELLULARIZED NERVE ALLOGRAFTS**
ENTZELLULÄRISIERTE NERVENALLOTRANSPLANTATE
ALLOGREFFES DE NERF DÉCELLULARISÉ

(30) Priority: 26.05.2015 US 201562166432 P
(43) Date of publication of application: 11.04.2018
(73) Proprietor: Mayo Foundation for Medical Education and Research, Rochester, MN 55905 (US)
(72) Inventor: GIUSTI, Guilherme, Wilmette, IL 60091 (US); BISHOP, Allen, T., Rochester, MN 55902 (US); SHIN, Alexander, Y., Rochester, MN 55906 (US); HUNDEPOOL, Caroline, A., 3062 GM Rotterdam (NL)
(74) Representative: Kunz, Herbert
(86) International application number: PCT/US2016/031313
(87) International publication number: WO 2016/191073

(56) References cited:
- WO-A1-2014/114043
- CA-A1- 2 886 815
- US-A1- 2008 077 251
- US-A1- 2009 306 790
- US-A1- 2010 268 336
- AMY M. MOORE ET AL: "Acellular nerve allografts in peripheral nerve regeneration: A comparative study", MUSCLE AND NERVEMUSCLE, vol. 44, no. 2, 9 June 2011 (2011-06-09), pages 221-234, XP55279489, ISSN: 0148-639X, DOI: 10.1002/mus.22033
- D. NEUBAUER ET AL: "Chondroitinase treatment increases the effective length of acellular nerve grafts", EXPERIMENTAL NEUROLOGY, ELSEVIER, AMSTERDAM, NL, vol. 207, no. 1, 31 August 2007 (2007-08-31), pages 163-170, XP022227033, ISSN: 0014-4886, DOI: 10.1016/J.EXPNEUROL.2007.06.006
- MOORE ET AL.: 'Acellular Nerve Allografts in Peripheral Nerve Regeneration: A Comparative Study.' MUSCLE & NERVE vol. 44, no. 2., August 2011, pages 221 - 234, XP055279489

## Description

### CROSS-REFERENCE TO RELATED APPLICATIONS

This application claims priority to U.S. Application Serial No. 62/166,432, filed on May 26, 2015.

### BACKGROUND

### 1. Technical Field

This document relates to decellularized nerve allografts. For example, this document provides methods and materials for using decellularized nerve allografts to repair nerve injuries or bridge a severed nerve, thereby restoring motor function of the nerve.

### 2. Background Information

Traumatic injuries to peripheral nerves can cause considerable disability and economic burden (Jaquet et al., J. Trauma, 51(4):687-92 (2001)). Although highly prevalent in military conflicts, peacetime injuries commonly result from trauma secondary to motor vehicle accidents, penetrating trauma, industrial injuries, and falls. It is estimated that 5% of the patients admitted to Level I trauma centers have a peripheral nerve injury (Noble et al., J. Trauma, 45(1):116-22 (1998); Taylor et al., Am. J. Phys. Med. Rehab., 87(5):381-5 (2008); and Campbell et al., Connecticut Med., 73(7):389-94 (2009)). The treatment of peripheral nerve injuries is dependent on mechanism of injury, time between injury and treatment, and concomitant injuries. The majority of peripheral nerve injuries require surgical reconstruction to restore sensation and function (Kovachevich et al., J. Hand Surg., 35(12):1995-2000 (2010)). For nerve injuries that cannot be directly repaired, a bridging nerve graft is necessary.

The gold standard for nerve reconstruction is the autograft (Millesi, Clin. Plast. Surg., 11(1):105-13 (1984)). Typically, the sural nerve is harvested and sectioned into cables to fit the diameter and length of the defect (Berger and Millesi, Clin. Orthop. Relat. Res., 133:49-55 (1978)). However, the use of the autograft is limited by supply, diameter, and length, and is accompanied by associated donor site morbidity (IJmpa et al., Annals Plast. Surg., 57(4):391-5 (2006)). This has constrained the ability to optimally reconstruct nerves of patients with multiple segmental defects where length of nerve graft needed far exceeds the availability and results in the need to prioritize the nerves to be reconstruct.

Moore et al. describe in Muscle & Nerve 44(2), 221-234 (2011), acellular nerve allografts in peripheral nerve regeneration.

### SUMMARY

The present invention is defined by the independent claims. The dependent claims depict additional embodiments of the invention.
This document relates to decellularized nerve allografts. For example, this document provides decellularized nerve allografts and methods and materials for using decellularized nerve allografts to repair nerve injuries or bridge a severed nerve. As described herein, decellularized nerve allografts that are prepared using elastase and stored under cold conditions (e.g., about 2 to 6 °C) without being frozen can be used to repair nerve injuries or bridge a severed nerve in a manner that restores motor function of the nerve. This restored motor function can be evident at 12 weeks, 16 weeks, 20 weeks, or longer following nerve reconstruction. Having the ability to repair injured or severed nerves in a manner that restores motor function using the methods and materials provided herein can allow surgeons and patients to minimize the disabilities and economic burden associated with nerve injuries.

In general, one aspect of this document features a composition for use in reconstructing an injured or severed nerve in a mammal. The composition comprises, or consists essentially of, a decellularized nerve graft of the same species as the mammal, wherein the decellularized nerve graft was prepared by contacting nerve tissue with from about 0.01 units/mL to about 1 unit/mL (e.g., about 0.05 units/mL) of elastase for at least about four hours (e.g., about 16 hours) to prepare the decellularized nerve graft, wherein the decellularized nerve graft was not frozen and was stored under cold conditions of from about 1.5 °C to about 6.5 °C, and wherein the decellularized nerve graft into the mammal to repair the injured or severed nerve, wherein motor function of the nerve is observed after the implanting of the decellularized nerve graft. The mammal can be a human. The function of the nerve can be observed six months after the implanting of said decellularized nerve graft. The decellularized nerve graft can be prepared by contacting nerve tissue with from about 0.03 units/mL to about 0.07 units/mL of elastase. The decellularized nerve graft can be prepared by contacting nerve tissue with from about 0.03 units/mL to about 0.07 units/mL of elastase for from about 10 hours to about 20 hours. The decellularized nerve graft can be one that was stored under cold conditions of from about 3 °C to about 5 °C.

In another aspect, this document features a method for making a decellularized nerve graft. The method comprises, or consists essentially of, (a) providing a nerve tissue from a mammal, and (b) contacting the nerve tissue with from about 0.01 units/mL to about 1 unit/mL (e.g., about 0.05 units/mL) of elastase and from about 0.5 units/mL to about 5 units/mL (e.g., about 2 units/mL) of a chondroitin-sulfate-ABC endolyase to form the decellularized nerve graft, wherein, when the decellularized nerve graft is implanted into a member of the same species as the mammal to repair an injured or severed nerve of the member, motor function of the injured or severed nerve is observed after being implanted. The mammal can be a human. The motor function of the injured or severed nerve can be observed six months after being implanted. The nerve tissue can be contacted with the elastase for from about 10 hours to about 20 hours (e.g., about 16 hours). The nerve tissue can be contacted with from about 0.03 units/mL to about 0.07 units/mL of elastase. The decellularized nerve graft can be one that was not frozen. The decellularized nerve graft can be one that was stored under cold conditions of from about 1.5 °C to about 6.5 °C.

In another aspect, this document features a decellularized nerve graft produced by contacting nerve tissue obtained from a mammal with from about 0.01 units/mL to about 1 unit/mL (e.g., about 0.05 units/mL) of elastase and from about 0.5 units/mL to about 5 units/mL (e.g., about 2 units/mL) of a chondroitin-sulfate-ABC endolyase to form the decellularized nerve graft, wherein, when the decellularized nerve graft is implanted into a member of the same species as the mammal to repair an injured or severed nerve of the member, motor function of the injured or severed nerve is observed after being implanted. The mammal can be a human. The motor function of the injured or severed nerve can be observed six months after being implanted. The nerve tissue can be nerve tissue that was contacted with the elastase for from about 10 hours to about 20 hours (e.g., about 16 hours). The nerve tissue can be nerve tissue that was contacted with from about 0.03 units/mL to about 0.07 units/mL of elastase. The decellularized nerve graft can be one that was not frozen. The decellularized nerve graft can be one that was stored under cold conditions of from about 1.5 °C to about 6.5 °C.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict between the present specification and a reference mentioned herein, the present specification, including definitions, will control. In addition, the materials, methods, and examples are illustrative only and not intended to be limiting.

Other features and advantages of the invention will be apparent from the following detailed description, and from the claims.

### DESCRIPTION OF DRAWINGS

Figure 1. Group comparison for ankle angle contracture and CMAP (compound muscle action potential). Results are expressed as a percentage of the contralateral, normal side and are presented as mean ± standard deviation. *Indicates significance (p<0.05).
Figure 2. Group comparison for isometric tetanic force and muscle weight. Results are expressed as a percentage of the contralateral, normal side and are presented as mean ± standard deviation. *Indicates significance (p<0.05).
Figure 3. Group comparison of histomorphometry of nerve area, myelin area, number of axons, and total axon area at 16 weeks postoperative. Results are expressed as a percentage of the contralateral, normal side and are presented as mean ± standard deviation. *Indicates significance (p<0.05).
Figure 4. Histological transverse sections of the peroneal nerve at 20 X magnification. Autograft group at 16 weeks (left); cold-stored allograft at 16 weeks (*middle*); and freeze-stored allograft at 16 weeks (*right*). Nerve size was similar between the three groups.
Figure 5. Overview of different staining for Groups III, IV, and V.
Figure 6. Staining Axons. The axons of Groups III and IV, which were treated with elastase and visualized with S100 staining, revealed a decrease in S100 when treated with elastase.
Figure 7. Staining Immunogenicity. The immunogenicity of the nerve grafts, visualized with MHCI staining, revealed a decrease in MHCI staining for the nerve grafts treated with elastase.
Figure 8. Staining Structure. The structure score of the nerve grafts was not significantly influenced by the enzyme. Frozen storage of the grafts significantly reduced the score.
Figure 9. Scanning electron microscopic images of the nerve graft: (A) Native nerve, (B) Cold preserved nerve allograft (Group II), and (C) Frozen nerve allograft (Group IV).

### DETAILED DESCRIPTION

This document provides decellularized nerve allografts. For example, this document provides decellularized nerve allografts prepared using elastase as well as methods and materials for using decellularized nerve allografts to repair nerve injuries or bridge a severed nerve.

As described herein, using elastase to prepare a decellularized nerve allograft and storing the decellularized nerve allograft under cold conditions (e.g., about 2 to 6 °C) without freezing can result in a decellularized nerve allograft that, when implanted into a recipient to repair nerve injuries or bridge a severed nerve, restores motor function of the nerve. In some cases, a decellularized nerve allograft produced using elastase can exhibit reduce immunogenicity (e.g., reduced immunogenicity (MHC-I), a reduced number of Schwann cells (e.g., S 100-positive Schwann cells), and/or positive structural properties (e.g., basal lamina and overall structural properties).

Any appropriate mammal having an injured or severed nerve can be treated using a decellularized nerve allograft provided herein. For example, humans, monkeys, horses, pigs, dogs, cats, rabbits, mice, and rats having an injured or severed nerve can be treated using a decellularized nerve allograft provided herein.

Any appropriate nerve tissue can be obtained from a donor. Examples of nerve tissue that can be obtained from one member of a species (e.g., a human) to create a decellularized nerve allograft for implantation into another member of that same species include, without limitation, peripheral nerve tissues. In some cases, the length of nerve tissue obtained from a donor is selected based on the length of the nerve injury being treated. For example, when a 15 mm section of damaged or severed nerve is to be repaired, then a section at least about 20 mm can be obtained from a donor.

Once obtained, the fresh nerve tissue can be processed to create a decellularized nerve allograft. Briefly, nerve segments can be immediately after harvest placed in RPMI 1640 solution at 4°C overnight. The next day, the nerve tissues can be placed in deionized distilled water. After about 8 hours, the water can be replaced by a solution containing about 125 mM sulfobetaine-10 (SB-10), about 10 mM phosphate, and about 50 mM sodium. The nerves can be agitated for about 15 hours and rinsed for about 15 minutes in a washing solution of about 10 mM phosphate and about 50 mM sodium. Next, the washing solution can be replaced by a solution containing about 0.14% Triton X-200, about 0.6 mM sulfobetaine-16 (SB-16), about 10 mM phosphate, and about 50 mM sodium and agitated for about 24 hours. Next, the tissues can be rinsed with a washing solution containing about 50 mM phosphate and about 100 mM sodium. The washing solution can be replaced by an SB-10 solution, and the nerves can be agitated for about 8 hours. Next, the nerves can be washed with a washing solution once and put into a solution of SB-16/Triton X-200. The nerves can be agitated for about 15 hours and then washed in a solution containing about 10 mM phosphate and about 50 mM sodium. At this point, the nerves can be incubated in a solution containing about 2 U/mL chondroitinase ABC for about 16 hours at room temperature and then washed in a solution containing about 10 mM phosphate and about 50 mM sodium. In some cases, 1-3 U/mL of chondroitinase ABC can be used. Then, nerve segments can be incubated in a solution containing about 0.05 U/mL elastase at about 37°C for about 16 hours. After that, the nerves can be sterilized with gamma radiation of about 2.5 kGray.

Once prepared, the decellularized nerve allograft can be stored under cold conditions (e.g., about 2 to 6 °C, about 3 to 5 °C, or about 4 °C) without freezing until the time of implantation. In some cases, a decellularized nerve allograft can be stored under cold conditions (e.g., about 2 to 6 °C, about 3 to 5 °C, or about 4 °C) without freezing for about 8 hours to about one month prior to being implanted into a recipient. Any appropriate surgical technique can be used to prepare the recipient's existing injured or severed nerve for repair with a decellularized nerve allograft provided herein. In general, an injured or severed nerve is repaired by removing scarred injured nerve ends to fresh nerve, placing a decellularized nerve allograft between the freshened nerve ends, and sewing them in place with microsutures or by using fibrin glue or by another coaptation method. Once implanted, the decellularized nerve allograft can restore motor function to the nerve being repaired. This restored motor function can be evident by 6 months or longer, depending on the distance of the nerve injury from the end organ.

The invention will be further described in the following examples, which do not limit the scope of the invention described in the claims.

### EXAMPLES

### Example 1 - Return of motor function using a decellularized nerve allograft Animals

66 Lewis rats (weighing 250-300 grams) were used, and 22 Sprague Dawley rats served as full major histocompatibility complex mismatch nerve donors. Lewis rats were used as they are known for their reduced tendency for autotomy (Carr et al., Annals Plast. Surg., 28(6):538-44 (1992)). Animals were randomly divided into three groups each treated for a 10 mm sciatic nerve gap. Group I (n=22) had a unilateral nerve gap reconstructed with a processed nerve allograft that was cold stored ('Allograft Cold'). Group II (n=22) had a similar procedure except the processed allograft was freeze-stored ('Allograft Frozen'). Group III (n=22) served as a control using a nerve autograft. The rats were given food and water *ad libitum* and were individually housed with a 12 hour light-dark cycle.

### Nerve processing

Twenty-two Sprague-Dawley rats, weighing 300-350 grams, were sacrificed with an intraperitoneal injection of pentobarbital after which 15 mm nerve segments were harvested aseptically. Nerve allografts were prepared as follows. Briefly, nerve segments were placed in RPMI medium (Roswell Park Memorial Institute), followed by subsequent steps with different detergents, two enzymatic steps, and gamma irradiation. In particular, nerves segments were immediately after harvest placed in RPMI 1640 solution at 4°C overnight. The next day, the nerve tissues were placed in deionized distilled water. After 8 hours, the water was replaced by a solution containing 125 mM sulfobetaine-10 (SB-10), 10 mM phosphate, and 50 mM sodium. The nerves were agitated for 15 hours and rinsed for 15 minutes in a washing solution of 10 mM phosphate and 50 mM sodium. Next, the washing solution was replaced by a solution containing 0.14% Triton X-200, 0.6 mM sulfobetaine-16 (SB-16), 10 mM phosphate, and 50 mM sodium, and the nerves agitated for 24 hours. Next, the tissues were rinsed with the washing solution containing 50 mM phosphate and 100 mM sodium. The washing solution was replaced by SB-10 solution, and the nerves were agitated for 8 hours. Next, they were washed with the washing solution once and put into a solution of SB-16/Triton X-200. The nerves were agitated for 15 hours and then washed in a solution containing 10 mM phosphate and 50 mM sodium. Subsequently, nerves were incubated in a solution containing 2 U/mL chondroitinase ABC for 16 hours at room temperature and then washed in a solution containing 10 mM phosphate and 50 mM sodium. Subsequently, nerve segments were incubated in a solution containing 0.05 U/mL elastase at 37°C for 16 hours. After that, the nerves were sterilized with gamma radiation of 2.5 kGray.

At the end, the processed allografts were either stored in phosphate buffered saline (PBS) at 4°C ('allograft cold') or stored in Ringers Lactate at -80°C ('allograft frozen') for 14 days.

### Surgical procedure

Rats were anesthetized with an intraperitoneal cocktail of ketamine (Ketaset®, 100 mg/mL; Fort Dodge Animal Health, Fort Dodge, IA) and xylazine (Vettek™, 100 mg/mL, Bluesprings, MO) 10:1 mixture, respectively at a dose of 1 mL/kg body weight after initial induction with Isoflurane. Anesthesia was maintained with additional doses of ketamine only. Ringer's lactate solution was administrated subcutaneously to prevent dehydration, and body temperature was maintained with a heating pad. The left sciatic nerve was exposed with a mid-gluteal incision. A 10 mm sciatic nerve segment was excised under an operating microscope (Zeiss OpMi6, Carl Zeiss Surgical GmbH, Oberkochen, Germany). In group I and II, a 10 mm nerve allograft cold or frozen, respectively, was used to bridge the 10 mm nerve gap using 10-0 nylon epineural sutures. In the control group (group III), the nerve segment was reversed and put back. The muscle was approximated with 5-0 Vicryl rapid sutures, and the skin was closed with the same sutures. Postoperatively, trimethoprim/sulfadiazine 30 mg/kg (Tribrissen, Five Star Compounding Pharmacy, Clive, IA) was administered to prevent infection, and buprenorphine (Buprinex®, 0.1 mL/kg, Reckitt Benckiser Pharmaceuticals Inc., Richmond, VA) served as an analgesic.

The motor functional outcome and histomorphometry of the nerve was tested at 12 and 16 weeks. During both time points, eleven animals per group were tested. The passive ankle angle, compound muscle action potential (CMAP), isometric tetanic force, and wet muscle weight were tested bilaterally. Distal nerve segments were analyzed for histomorphometry.

### Evaluation of Motor Functional Outcome

For the sacrificial procedure, at 12 and 16 weeks, the animals were anesthetized.

### Ankle Angle

The maximum passive plantar flexion angle of the ankle was measured bilaterally in all animals to determine the ankle contracture angle as described elsewhere (Lee et al., Plast. Reconstr. Surg., 132(5):1173-80 (2013)).

### Electrophysiology

The sciatic nerve was exposed. A miniature bipolar electrode (Harvard Apparatus, Holliston, MA) was attached proximal to the nerve graft. Recording electrodes were placed subcutaneous to the tibialis anterior muscle, and a ground electrode was placed in the surrounding tissue. Compound muscle action potential (CMAP) was measured using an EMG (VikingQuest, Nicolet Biomedical, Madison, WI). The maximal amplitude was recorded. In a similar fashion, the contralateral side was measured. The skin was re-approximated upon further testing.

### Maximum Isometric Tetanic Force

For obtaining the maximum isometric tetanic force, the peroneal nerve, distal of the nerve graft, was exposed. The force measurements were executed as described elsewhere (Shin et al., Microsurgery, 28(6):452-7 (2008)). Briefly, the tibial muscle was carefully freed from its insertion while preserving the neurovascular pedicle. The hindlimb was stabilized with two Kirschner wires (DePuy Orthopedics) in the distal femur and ankle joint. The distal tendon of the tibial muscle was attached to a force transducer (MDB-0.5, Transducer Techniques, Temecula, CA) using a custom clamp with the tendon aligned in the anatomical position. The force transducer signal was processed and analyzed with a computer using LabView software (National Instruments). A miniature bipolar electrode (Harvard Apparatus) was attached to the peroneal nerve. The nerve was stimulated with a stimulator (Grass SD9, Grass Instrument Co., Quincy, MA). After establishing the optimal preload or muscle resting length, the stimulus intensity was increased until maximum tetanic muscle force was reached.

### Wet Muscle Mass

After bilateral force testing, animals were sacrificed with an overdose of pentobarbital intraperitoneally. The tibial muscles of both hindlimbs were carefully dissected and weighed immediately to obtain muscle mass ratio.

### Histomorphometry

Nerve segments of the peroneal nerve were excised and stored in Trumps solution (37% formaldehyde and 25% glutaraldehyde) and subsequently embedded in spur resin. 1 µm sections were cut and stained with 1% Toluidine Blue. Images were acquired with a camera (Eclipse 50i; Nikon instruments, Melville, New York) and analyzed using Image ProPlus Software (Media Cybernetics Inc, Bethesda, MD), where nerve area, total myelin area, number of axons, and total axon area were obtained in semi-automatic fashion.

### Statistical Methods

The sample size of the groups was based on the results of muscle force test obtained from previous studies showing the highest standard deviation being approximately 10%. Assuming that same variability will occur (two tailed distribution, α=0.05), the number of animals to provide an 80% power to detect 10% difference between the groups was estimated to be 19. To guard against potential attrition and to overpower the study, the sample size was increased to 22 per group. The three groups were compared with respect to ankle contracture, electrophysiology, maximum isometric tetanic force, wet muscle weight, and nerve histomorphometry. Data were expressed as a percentage of the contralateral (healthy) side to diminish intra-animal differences. One-way analysis of variance (ANOVA) followed by a Bonferroni correction for multiple testing was used for statistical analysis. All results are presented as mean ± Standard Deviation (SD). A p-value of 0.05 was considered significant.

### Results

All animals survived the surgical procedure, and no complications were observed. All animals were used for final analysis. A summary of all results is presented in Table

**Table 1. Summary of results of test for all groups.**

| | | Group I | | Group II | | Group III | |
|---|---|---|---|---|---|---|---|
| Surgical intervention | | Autograft | | Cold stored Allograft | | Frozen stored Allograft | |
| No. of animals tested | | 11 | 11 | 11 | 11 | 11 | 11 |
| Sacrifice time (wk) | | 12 | 16 | 12 | 16 | 12 | 16 |
| Maximum passive plantar flexion ankle angle (%) | | 80.2 ± 1.0 | 88.0 ± 0.9 | 73.7 ± 1.2 | 77.4 ± 1.1 | 73.1 ± 1.3 | 74.1 ± 0.1 |
| CMAP (%) | | 41.9 ± 4.6 | 44.0 ± 6.9 | 44.6 ± 4.8 | 56.2 ± 4.4 | 40.8 ± 1.7 | 53.5 ± 4.0 |
| Maximum isometric tetanic tension (%) | | 42.3 ± 1.8 | 53.9 ± 3.8 | 48.7 ± 2.4 | 55.4 ± 4.0 | 43.2 ± 3.2 | 50.0 ± 3.6 |
| Tibialis anterior wet muscle weight (%) | | 63.7 ± 1.5 | 71.1 ± 1.5 | 60.2 ± 1.5 | 67.0 ± 2.1 | 58.3 ± 1.3 | 64.7 ± 1.2 |
| Histomorphometry of peroneal nerve (%) | Nerve area: | 74.6 ± 4.9 | 92.2 ± 4.4 | 86.9 ± 5.9 | 78.7 ± 5.6 | 76.7 ± 3.6 | 71.4 ± 2.7 |
| | Myelin area: | 55.5 ± 4.1 | 72.8 ± 3.3 | 75.7 ± 6.9 | 66.1 ± 4.9 | 57.3 ± 3.2 | 53.4 ± 2.3 |
| | No. of axons: | 120.1 ± 11.5 | 130.0 ± 5.4 | 138.4 ± 9.5 | 130.3 ± 10.4 | 101.8 ± 4.7 | 101.0 ± 6.6 |
| | Axon area: | 29.8 ± 3.7 | 35.0 ± 4.0 | 30.7 ± 3.0 | 35.0 ± 3.1 | 18.9 ± 11.3 | 23.6 ± 1.5 |

### Ankle Angle

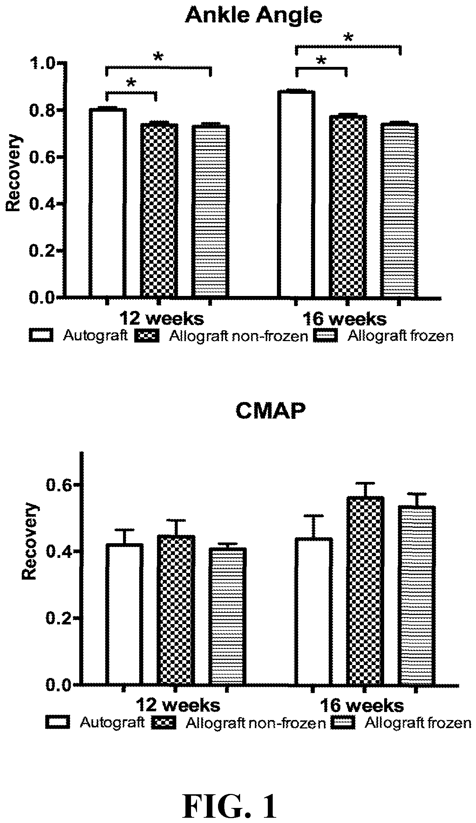

The percentage of recovery of the ankle angle contracture of the experimental side compared to the contralateral side was 80.2 ± 3.1 % in group I, 73.7 ± 3.9 % in group II, and 73.1 ± 4.2 % in group III at 12 weeks. At 16 weeks, the recovery was 88.0 ± 3.1 % in group I, 77.4 ± 3.6 % in group II, and 74.1 ± 3.1 % in group III. Significant difference was observed between group I and III (p<0.001) at both 12 and 16 weeks postoperatively (Figure 1).

### Electrophysiology

Recovery of the compound muscle action potentials (CMAP) at 12 weeks was 41.9 ± 14.4 % in group I, 44.5 ± 15.1 % in group II, and 40.8 ± 5.3 % in group III. At 16 weeks, the recovery increased to 44.0 ± 21.9 % in group I, 56.2 ± 14.0 % in group II, and 53.5 ± 12.7 % in group III. Group comparison showed no statistically significant difference between all groups at both time points (Figure 1).

### Isometric Tetanic Force

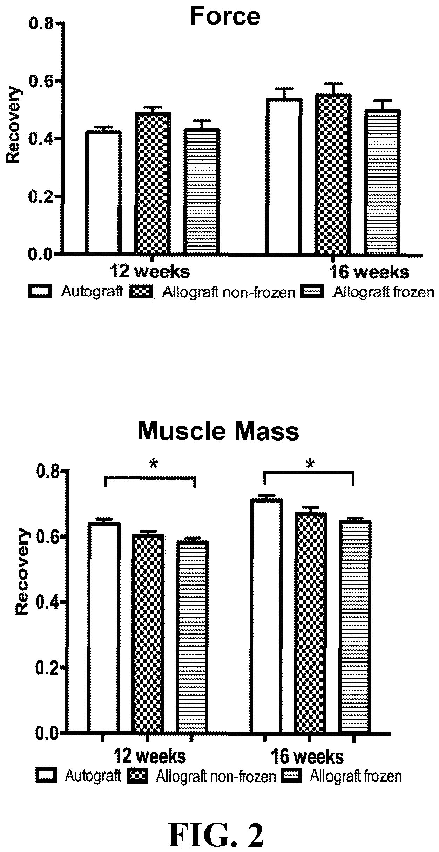

In group I, the percentage of muscle force recovery was found to be 42.3 ± 5.8 %. Group II exhibited the highest recovery with 48.7 ± 7.6 %. In group III, it was 43.2 ± 10.1 %. In the late follow-up time, at 16 weeks, the muscle force was recovered to 53.9 ± 12.0 % in group I, 55.4 ± 12.7 % in group II, and 50.0 ± 11.4 % in group III. No statistical significant difference was found when the groups were compared at the early and late follow up times (Figure 2).

### Muscle Weight

The muscle mass ratio of the tibial muscle at 12 weeks was 63.7 ± 4.9 %, 60.2 ± 4.7 %, and 58.3 ± 4.1 % for groups I, II, and III, respectively. At 16 weeks, the muscle weight revealed a recovery up to 71.1 ± 4.8 % in group I, 67.0 ± 6.6 % in group II, and 64.7 ± 3.7 % in group III. At both time points, a statistically significant difference was observed between groups I and III (p < 0.05 at both weeks). The autograft performed better than the frozen allograft. No difference was found when comparing the autograft to the cold allograft (Figure 2).

### Histomorphometry

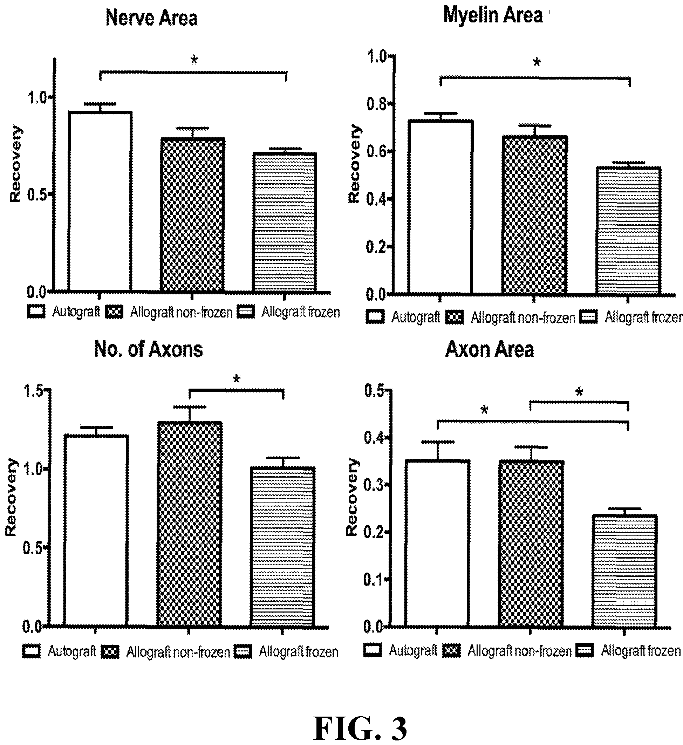

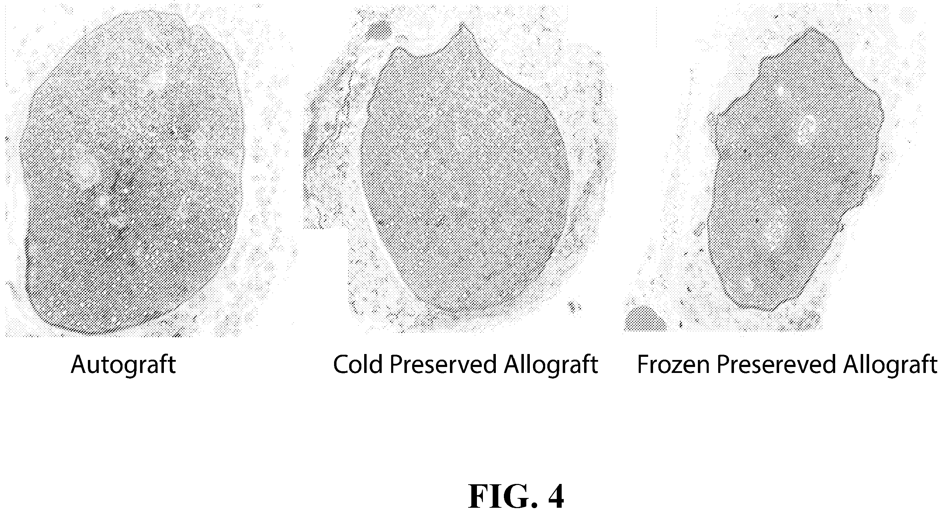

Figure 3 shows the normalized results of the histomorphometry of the different groups 16 weeks postoperatively. Total nerve area, myelin area, and axon area were statistically lower in the frozen allograft group when compared to the autograft (p<0.05). The cold stored allograft did not significantly differ from the nerve autograft (Figures 3 and 4).

### Example 2 - Decellularization techniques to create a nerve allograft

Twenty-five Sprague-Dawley rats, weighing 250-350 grams (Harlan, Indianapolis, IN), were used. After initial Isoflurane induction, all animals were sacrificed with an overdose of pentobarbital. Bilateral, 15 mm nerve segments of the sciatic nerve were aseptically harvested. A total of 50 nerve segments were collected.

### Experimental design

A total of 5 groups were compared in this study. All groups consisted of 10 nerves. The first group was processed following a standard protocol based on previous studies (Hudson et al., Tissue Engineering, 10(9-10):1346-58 (2004); Neubauer et al., Exp. Neurol., 207(1): 163-70 (2007); and Giusti et al., J. Bone Joint Surg. Am., 7;94(5):410-7 (2012)). The second and third group underwent the same processing only with the addition of the enzyme elastase in two different time periods (i.e., 8 and 16 hours; Group II and III, respectively). The effect of freeze storage (-80°C) was also studied in Group IV. A native unprocessed nerve (Group V) was analyzed as a negative control.

An overview of the studied groups is depicted in Table 2.

**Table 2. Experimental design.**

| **Group** | **Treatment** | **Storage** |
|---|---|---|
| I | Standard | Cold (4 °C) |
| II | Standard + Elastase (short) | Cold (4 °C) |
| III | Standard + Elastase (long) | Cold (4 °C) |
| IV | Standard + Elastase (long) | Freeze (-80 °C) |
| V | Un-processed/native nerve | No |

### Nerve allograft processing

Briefly, nerves segments were immediately after harvest placed in RPMI 1640 solution at 4 °C overnight. The next day, the nerve tissues were placed in deionized distilled water. After 8 hours, the water was replaced by a solution containing 125 mM sulfobetaine-10 (SB-10), 10 mM phosphate, and 50 mM sodium. The nerves were agitated for 15 hours and rinsed for 15 minutes in a washing solution of 10 mM phosphate and 50 mM sodium. Next, the washing solution was replaced by a solution containing 0.14% Triton X-200, 0.6 mM sulfobetaine-16 (SB-16), 10 mM phosphate, and 50 mM sodium and agitated for 24 hours. Next, the tissues were rinsed with the washing solution of 50 mM phosphate and 100 mM sodium. The washing solution was replaced by SB-10 solution, and the nerves were agitated for 8 hours. Next, they were washed once using the washing solution and put into a solution of SB-16/Triton X-200. The nerves were agitated for 15 hours and then washed in a solution containing 10 mM phosphate and 50 mM sodium. Subsequently, nerves were incubated in a solution containing 2 U/mL Chondroitinase ABC for 16 hours at room temperature and then washed in a solution containing 10 mM phosphate and 50 mM sodium. In the Groups 2-4, nerves, which underwent elastase treatment, were incubated in a solution containing 0.05 U/mL elastase at 37 °C for 8 hours (Group II) or 16 hours (Groups III and IV). After that, the nerves were sterilized with gamma radiation of 2.5 kGray.

### Storage

Nerve segments were stored in Ringers solution at -80°C for the freeze storage (Group IV) for a duration of two weeks before final analysis. The other storage method was cold storage (4°C), where nerves were placed in PBS solution.

### Outcome analysis

### Structure

A 5 mm section of each nerve segment was fixed in 2% Trump's solution (37% formaldehyde and 25% glutaraldehyde). 1 µm thin sections were transversally cut and stained with 1% toluidine blue. Another 5 mm section of each nerve segment was suspended in OCT and fast frozen, and 5 µm transverse sections were cut. Nerve sections were stained with hematoxylin and eosin (H&E). Digital images of each sample were taken using a microscope digital camera (Nikon microscopy digital color camera 4.0 mega pixels, Melville, New York).

The organization of the basal lamina was visualized with a laminin staining as described herein. For electron microscopy, ultra-thin section were cut (500 A), placed on copper grids (200 mesh, EMS, Philadelphia, PA) and stained with uranyl acetate (EMS) and lead citrate (EMS). Sections were examined under a JEOL 1400 transmission electron microscope (JEOL Ltd, Peabody, Massachusetts, USA). All sections were scored for their structural properties on a 1-5 scale with 1 being worst and 5 being optimal. Three independent and blinded investigators performed the analysis. Validity and reliability of the objective analysis was determined with an intra-class correlation of 0.83 (95% CI; 0.71-0.90).

### Remnants (Axons and Immunogenicity)

Intraluminal remnants were examined with immunohistochemical (IHC) stainings on different components of the nerve allograft. Nerve segments were pre-fixed with 4% cold paraformaldehyde and fast frozen. Transverse frozen sections (5 µm thickness) were cut. To identify the remnant axons left in the graft, a S100 staining was performed. To study the immunogenicity of the graft after processing, MHC-I was stained. Additionally, laminin was stained to identify the basal laminae. The IHC staining procedure was performed using the Leica Bond III Stainer (Leica, Buffalo, IL). The sections were post fixed in 4% paraformaldehyde and retrieved on-line using Epitope Retrieval 1 (Leica, Buffalo, IL) for 5 minutes. The following primary antibodies were used: polyclonal S100 anti-rabbit (Dako) was used at 1:5000, polyclonal laminin yl anti-rabbit (Sigma) was used at 1:200, and mouse anti-MHCI (Clone OX18, Novus Biological) was used at 1:100. All antibodies were incubated for 60 minutes. The detection system used was Research detection (Leica DS9455). This system includes the Protein Block (Dako X0909) and secondary antibody AlexFluor488. All sections were nuclear stained with Hoechst33342 (Invitrogen H1399).

Once completed, slides were removed from stainer and rinsed for 5 minutes in distilled water. Slides were coverslipped using ProLong Gold antifade media (Invitrogen). Nerve slides were examined under a fluorescence laser confocal microscope (LSM 780, Zeiss, Germany), and pictures were captured with a camera. The intensity of stainings in the cross section of the nerve was measured with Image J software (NIH, Bethesda, USA).

### Statistical Analysis

Data were expressed as mean ± SEM. For structural analysis, the results of the three different observers and the three different stainings were averaged to score the structural properties. Statistical analysis of the differences between the groups was performed with one-way analysis of variance (ANOVA) followed by Bonferroni post hoc test with GraphPad Prism 5 software (GraphPad Software, CA, USA). P-values < 0.05 were considered to be significant.

### Results

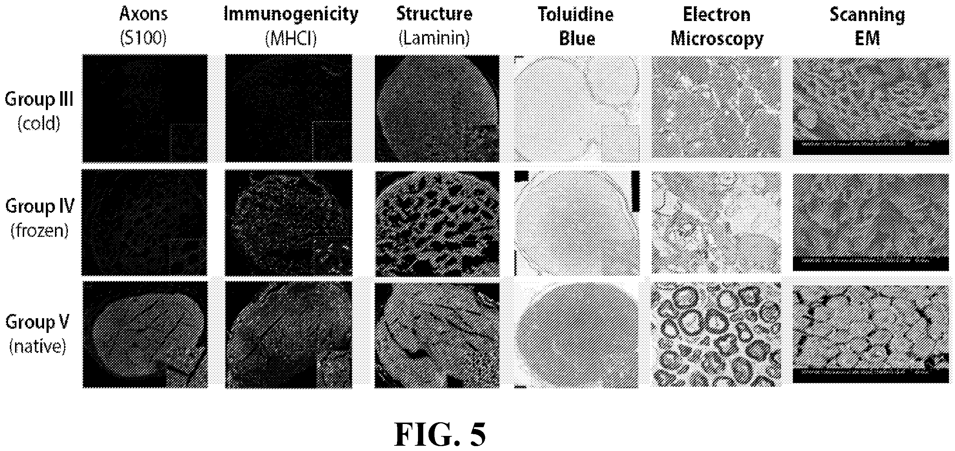

An overview of the different stainings is depicted in Figure 5, and a summary of the results is provided in Table 3.

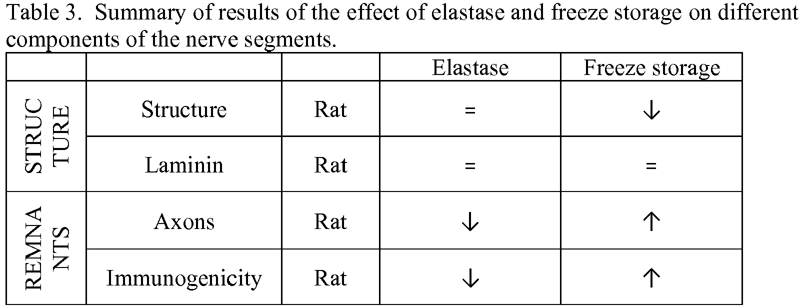

### Enzymatic decellularization

### Structure: score

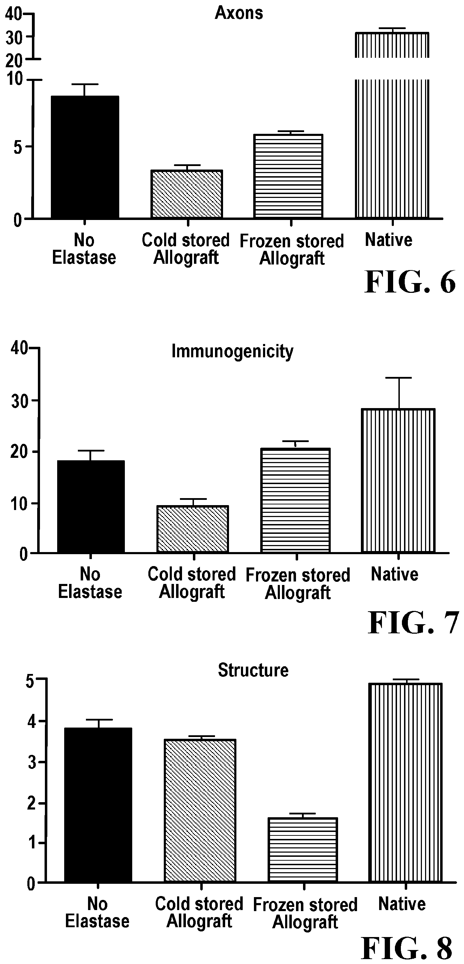

The structure of the nerve graft was not significantly influenced by the addition of elastase to the decellularization protocol. Group I, the standard protocol, exhibited a score of 3.9 ± 0.2, and Group II, with the addition of elastase, exhibited a score of 3.5 ± 0.1. There was no significant difference between the groups. A longer exposure of the enzyme, Group III, did not influence the structure of the nerve graft (3.2 ± 0.1). There was no statistical difference between the groups (Figure 6).

### Structure: Laminin

The same was observed when the intensity of the laminin was determined. The enzyme did not significantly reduce the presence of laminin in the nerve. Group I (standard protocol) had a laminin intensity of 65.7 ± 8.6, Group II (with elastase) had a laminin intensity of 53.22 ± 6.2, and the group with a longer exposure to elastase, Group III, had a score of 51.5 ± 3.3. No significant difference between those three groups was observed (p=0.20).

### Remnants: Axons

Axons were significantly reduced by the addition of the extra enzymatic step (Group I; 9.5 ± 1.0, Group II; 5.0 ± 0.5). Prolonged exposure to elastase (Group III) resulted in an even lower score of axons (3.2 ± 0.4). The difference between the groups was statistically significant (p<0.0001) (Figure 7).

### Remnants: Immunogenicity

Elastase had a similar significant effect of reduction of the remnants when evaluating immunogenicity. The standard protocol without the enzyme (Group I) had an MHCI score of 18.21 ± 1.8, and the addition of elastase reduced the MHCI score to 9.3 ± 1.0. The observed differences were significant, p<0.0001 (Figure 8).

### Storage

### Structure: score

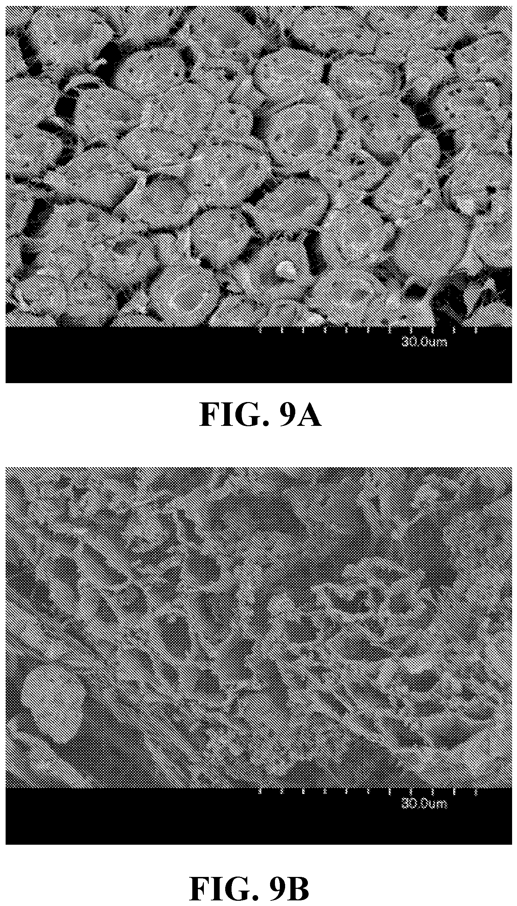

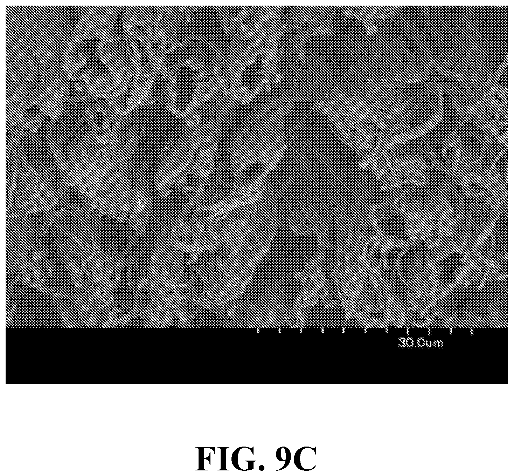

The effect of cold or freeze storage at either 4°C or -80°C revealed a tremendous effect on the structure of the graft. When frozen, the total score for the structure significantly decreased from 3.2 ± 0.1 (Group III) to 1.6 ± 0.2 (Group IV) for the rat nerves. The effect of storage was visualized with electron microscopy in Figure 9.

### Structure: Laminin

Storage had no significant effect on the laminin intensity staining. The score of Group III (51.49 ± 3.31) was not significantly different compared to that of Group IV (63.67 ± 2.4, p=0.20).

### Remnants: Axons

When examining the effect of storage on the intraluminal remnants, the cold storage nerves exhibited a significantly lower amount of axons, stained with S100 compared to the frozen nerves. The effect of storage was statistically significant. Group III was 3.2 ± 0.3, and the freeze storage group (Group IV) was higher at 5.7 ± 0.3 (Figure 6).

### Remnants: Immunogenicity

The effect of cold or freeze storage at either 4°C or -80°C revealed a severe effect on the structure of the graft. The immunogenicity in the rat nerves of the cold storage group (Group III), stained with MHCI, was statistically significant different (9.3 ± 1.0) from the frozen storage (Group IV; 20.8 ± 1.2) (Figure 7).

The results provided herein demonstrate a reduced immunogenicity, diminished cellular debris, and elimination of Schwann cells, while maintaining ultrastructure, when elastase was added to the nerve processing. Storage at -80°C after the decellularization process heavily damaged the nerve ultrastructure as compared to cold storage.

## Claims

1. A composition for use in reconstructing an injured or severed nerve in a mammal, wherein said composition comprises:
a decellularized nerve graft of the same species as said mammal, wherein said decellularized nerve graft was prepared by contacting nerve tissue with from 0.01 units/mL to 1 unit/mL of elastase for at least four hours to prepare said decellularized nerve graft, wherein said decellularized nerve graft was not frozen and was stored under cold conditions of from 1.5 °C to 6.5 °C, and
wherein said decellularized nerve graft is to be implanted into said mammal to repair said injured or severed nerve, wherein motor function of said nerve is observed after said implanting of said decellularized nerve graft.

2. The composition for use of claim 1, wherein said mammal is a human.

3. The composition for use of any one of claims 1-2, wherein said function of said nerve is observed six months after said implanting of said decellularized nerve graft.

4. The composition for use of any one of claims 1-3, wherein said decellularized nerve graft was prepared by contacting nerve tissue with from 0.03 units/mL to 0.07 units/mL of elastase, optionally wherein said decellularized nerve graft was prepared by contacting nerve tissue with from 0.03 units/mL to 0.07 units/mL of elastase for from 10 hours to 20 hours.

5. The composition for use of any one of claims 1-4, wherein said decellularized nerve graft was stored under cold conditions of from 3 °C to 5 °C.

6. A method for making a decellularized nerve graft, wherein said method comprises:
(a) providing a nerve tissue from a mammal, and
(b) contacting said nerve tissue with from 0.01 units/mL to 1 unit/mL of elastase and from 0.5 units/mL to 5 units/mL of a chondroitin-sulfate-ABC endolyase to form said decellularized nerve graft,
wherein, when said decellularized nerve graft is implanted into a member of the same species as said mammal to repair an injured or severed nerve of said member, motor function of said injured or severed nerve is observed after being implanted.

7. The method of claim 6, wherein said mammal is a human.

8. The method of any one of claims 6-7, wherein said motor function of said injured or severed nerve is observed six months after being implanted.

9. The method of any one of claims 6-8, wherein said nerve tissue is contacted with said elastase for from 10 hours to 20 hours, optionally wherein said nerve tissue is contacted with from 0.03 units/mL to 0.07 units/mL of elastase.

10. The method of any one of claims 6-9, wherein said decellularized nerve graft is not frozen, optionally wherein said decellularized nerve graft is stored under cold conditions of from 1.5 °C to 6.5 °C.

11. A decellularized nerve graft produced by contacting nerve tissue obtained from a mammal with from 0.01 units/mL to 1 unit/mL of elastase and from 0.5 units/mL to 5 units/mL of a chondroitin-sulfate-ABC endolyase to form said decellularized nerve graft, wherein, when said decellularized nerve graft is implanted into a member of the same species as said mammal to repair an injured or severed nerve of said member, motor function of said injured or severed nerve is observed after being implanted.

12. The decellularized nerve graft of claim 11, wherein said mammal is a human.

13. The decellularized nerve graft of any one of claims 11-12, wherein said motor function of said injured or severed nerve is observed six months after being implanted.

14. The decellularized nerve graft of any one of claims 11-13, wherein said nerve tissue is contacted with said elastase for from 10 hours to 20 hours, optionally wherein said nerve tissue is contacted with from 0.03 units/mL to 0.07 units/mL of elastase.

15. The decellularized nerve graft of any one of claims 11-14, wherein said decellularized nerve graft was not frozen, optionally wherein said decellularized nerve graft was stored under cold conditions of from 1.5 °C to 6.5 °C.

## Patentansprüche

1. Zusammensetzung zur Verwendung bei der Rekonstruktion eines verletzten oder durchtrennten Nervs bei einem Säuger, wobei die Zusammensetzung umfasst:
ein entzellularisiertes Nerventransplantat derselben Spezies wie der Säuger, wobei das entzellularisierte Nerventransplantat gewonnen wurde, indem Nervengewebe mindestens vier Stunden lang mit 0,01 Einheiten/ml bis 1 Einheit/ml Elastase in Kontakt gebracht wurde, um das entzellularisierte Nerventransplantat herzustellen, wobei das entzellularisierte Nerventransplantat nicht eingefroren war und unter kalten Bedingungen von 1,5 °C bis 6,5 °C aufbewahrt wurde, und
wobei das entzellularisierte Nerventransplantat in den Säuger implantiert werden soll, um den verletzten oder durchtrennten Nerv zu reparieren, wobei die motorische Funktion des Nervs nach der Implantation des entzellularisierten Nerventransplantats beobachtet wird.

2. Zusammensetzung zur Verwendung nach Anspruch 1, wobei es sich bei dem Säuger um einen Menschen handelt.

3. Zusammensetzung zur Verwendung nach einem der Ansprüche 1 und 2, wobei die Funktion des Nervs sechs Monate nach der Implantation des entzellularisierten Nerventransplantats beobachtet wird.

4. Zusammensetzung zur Verwendung nach einem der Ansprüche 1 bis 3, wobei das entzellularisierte Nerventransplantat durch Inkontaktbringen von Nervengewebe mit 0,03 Einheiten/ml bis 0,07 Einheiten/ml Elastase gewonnen wurde, wobei das entzellularisierte Nerventransplantat optional durch Inkontaktbringen von Nervengewebe mit 0,03 Einheiten/ml bis 0,07 Einheiten/ml Elastase für 10 Stunden bis 20 Stunden gewonnen wurde.

5. Zusammensetzung zur Verwendung nach einem der Ansprüche 1 bis 4, wobei das entzellularisierte Nerventransplantat unter kalten Bedingungen von 3 °C bis 5 °C aufbewahrt wurde.

6. Verfahren zur Erzeugung eines entzellularisierten Nerventransplantats, wobei das Verfahren umfasst:
(a) Bereitstellen eines Nervengewebes von einem Säuger, und
(b) Inkontaktbringen des Nervengewebes mit 0,01 Einheiten/ml bis 1 Einheit/ml Elastase und 0,5 Einheiten/ml bis 5 Einheiten/ml einer Chondroitinsulfat-ABC-Endolyase, um das entzellularisierte Nerventransplantat zu bilden, wobei, wenn das entzellularisierte Nerventransplantat in ein Körperglied derselben Spezies wie der des Säugers implantiert wird, um einen verletzten oder durchtrennten Nerv des Körperglieds zu reparieren, die motorische Funktion des verletzten oder durchtrennten Nervs nach der Implantation beobachtet wird.

7. Verfahren nach Anspruch 6, wobei es sich bei dem Säuger um einen Menschen handelt.

8. Verfahren nach einem der Ansprüche 6 bis 7, wobei die motorische Funktion des verletzten oder durchtrennten Nervs sechs Monate nach der Implantation beobachtet wird.

9. Verfahren nach einem der Ansprüche 6 bis 8, wobei das Nervengewebe 10 bis 20 Stunden lang mit der Elastase in Kontakt gebracht wird, wobei das Nervengewebe optional mit 0,03 Einheiten/ml bis 0,07 Einheiten/ml Elastase in Kontakt gebracht wird.

10. Verfahren nach einem der Ansprüche 6 bis 9, wobei das entzellularisierte Nerventransplantat nicht eingefroren wird, wobei das entzellularisierte Nerventransplantat optional unter kalten Bedingungen von 1,5 °C bis 6,5 °C aufbewahrt wird.

11. Entzellularisiertes Nerventransplantat, erzeugt durch Inkontaktbringen von Nervengewebe, das von einem Säuger gewonnen wurde, mit 0,01 Einheiten/ml bis 1 Einheit/ml Elastase und 0,5 Einheiten/ml bis 5 Einheiten/ml einer Chondroitinsulfat-ABC-Endolyase zur Bildung des entzellularisierten Nerventransplantats, wobei, wenn das entzellularisierte Nerventransplantat in ein Körperglied derselben Spezies wie das des Säugers implantiert wird, um einen verletzten oder durchtrennten Nerv des Körperglieds zu reparieren, die motorische Funktion des verletzten oder durchtrennten Nervs nach der Implantation beobachtet wird.

12. Entzellularisiertes Nerventransplantat nach Anspruch 11, wobei es sich bei dem Säuger um einen Menschen handelt.

13. Entzellularisiertes Nerventransplantat nach einem der Ansprüche 11 bis 12, wobei die motorische Funktion des verletzten oder durchtrennten Nervs sechs Monate nach der Implantation beobachtet wird.

14. Entzellularisiertes Nerventransplantat nach einem der Ansprüche 11 bis 13, wobei das Nervengewebe 10 bis 20 Stunden lang mit der Elastase in Kontakt gebracht wird, wobei das Nervengewebe optional mit 0,03 Einheiten/ml bis 0,07 Einheiten/ml Elastase in Kontakt gebracht wird.

15. Entzellularisiertes Nerventransplantat nach einem der Ansprüche 11 bis 14, wobei das entzellularisierte Nerventransplantat nicht eingefroren wurde, wobei das entzellularisierte Nerventransplantat optional unter kalten Bedingungen von 1,5 °C bis 6,5 °C aufbewahrt wurde.

## Revendications

1. Composition pour utilisation dans la reconstruction d'un nerf blessé ou sectionné chez un mammifère, dans laquelle ladite composition comprend :
une greffe de nerf décellularisé de la même espèce que ledit mammifère, dans laquelle ladite greffe de nerf décellularisé a été préparée par mise en contact de tissu nerveux avec entre 0,01 unité/mL à 1 unité/mL d'élastase pendant au moins quatre heures pour préparer ladite greffe de nerf décellularisé, dans laquelle ladite greffe de nerf décellularisé n'a pas été congelée et a été stockée dans des conditions froides comprises entre 1,5 °C et 6,5 °C, et
dans laquelle ladite greffe de nerf décellularisé doit être implantée dans ledit mammifère pour réparer ledit nerf blessé ou sectionné, dans laquelle la fonction motrice dudit nerf est observée après ladite implantation de ladite greffe de nerf décellularisé.

2. Composition pour utilisation selon la revendication 1, dans laquelle ledit mammifère est un humain.

3. Composition pour utilisation selon l'une quelconque des revendications 1 à 2, dans laquelle ladite fonction dudit nerf est observée six mois après ladite implantation de ladite greffe de nerf décellularisé.

4. Composition pour utilisation selon l'une quelconque des revendications 1 à 3, dans laquelle ladite greffe de nerf décellularisé a été préparée par mise en contact du tissu nerveux avec entre 0,03 unité/mL et 0,07 unité/mL d'élastase, éventuellement dans laquelle ladite greffe de nerf décellularisé a été préparée par mise en contact de tissu nerveux avec entre 0,03 unité/mL et 0,07 unité/mL d'élastase pendant entre 10 heures et 20 heures.

5. Composition pour utilisation selon l'une quelconque des revendications 1 à 4, dans laquelle ladite greffe de nerf décellularisé a été stockée dans des conditions froides comprises entre 3 °C et 5 °C.

6. Procédé de fabrication d'une greffe de nerf décellularisé, dans lequel ledit procédé comprend :
(a) le fait de se munir d'un tissu nerveux provenant d'un mammifère, et
(b) la mise en contact dudit tissu nerveux avec entre 0,01 unité/mL et 1 unité/mL d'élastase et entre 0,5 unité/mL et 5 unités/mL d'une chondroïtine-sulfate-ABC endolyase pour former ladite greffe de nerf décellularisé,
dans lequel, lorsque ladite greffe de nerf décellularisé est implantée dans un membre de la même espèce que ledit mammifère pour réparer un nerf blessé ou sectionné dudit membre, la fonction motrice dudit nerf blessé ou sectionné est observée après son implantation.

7. Procédé selon la revendication 6, dans lequel ledit mammifère est un humain.

8. Procédé selon l'une quelconque des revendications 6 à 7, dans lequel ladite fonction motrice dudit nerf blessé ou sectionné est observée six mois après son implantation.

9. Procédé selon l'une quelconque des revendications 6 à 8, dans lequel ledit tissu nerveux est mis en contact avec ladite élastase pendant entre 10 heures et 20 heures, éventuellement dans lequel ledit tissu nerveux est mis en contact avec entre 0,03 unité/mL et 0,07 unité/mL d'élastase.

10. Procédé selon l'une quelconque des revendications 6 à 9, dans lequel ladite greffe de nerf décellularisé n'est pas congelée, éventuellement dans lequel ladite greffe de nerf décellularisé est stockée dans des conditions froides comprises entre 1,5 °C et 6,5 °C.

11. Greffe de nerf décellularisé produite par mise en contact de tissu nerveux obtenu auprès d'un mammifère avec entre 0,01 unité/mL et 1 unité/mL d'élastase et entre 0,5 unité/mL et 5 unités/mL d'une chondroïtine-sulfate-ABC endolyase pour former ladite greffe de nerf décellularisé, dans laquelle, lorsque ladite greffe de nerf décellularisé est implantée dans un membre de la même espèce que ledit mammifère pour réparer un nerf blessé ou sectionné dudit membre, la fonction motrice dudit nerf blessé ou sectionné est observée après son implantation.

12. Greffe de nerf décellularisé selon la revendication 11, dans laquelle ledit mammifère est un humain.

13. Greffe de nerf décellularisé selon l'une quelconque des revendications 11 à 12, dans laquelle ladite fonction motrice dudit nerf blessé ou sectionné est observée six mois après son implantation.

14. Greffe de nerf décellularisé selon l'une quelconque des revendications 11 à 13, dans laquelle ledit tissu nerveux est mis en contact avec ladite élastase pendant entre 10 heures et 20 heures, éventuellement dans lequel ledit tissu nerveux est mis en contact avec entre 0,03 unité/mL et 0,07 unité/mL d'élastase.

15. Greffe de nerf décellularisé selon l'une quelconque des revendications 11 à 14, dans laquelle ladite greffe de nerf décellularisé n'a pas été congelée, éventuellement dans laquelle ladite greffe de nerf décellularisé a été stockée dans des conditions froides comprises entre 1,5 °C et 6,5 °C.
